# FASCICULE DE BREVET EUROPEEN

(11) **EP 4 366 645 B1**
(45) Date de publication et mention de la délivrance du brevet: **22.04.2026**
(21) Numéro de dépôt: 22754460.8
(22) Date de dépôt: 05.07.2022
(51) Int. Cl.: A61B 34/20, A61B 17/17

(54) **SYSTÈME D'ASSISTANCE À LA FIXATION D'UN IMPLANT CHIRURGICAL DANS UN OS D'UN PATIENT**
HILFSSYSTEM ZUR BEFESTIGUNG EINES CHIRURGISCHEN IMPLANTATS IN EINEM KNOCHEN EINES PATIENTEN
SYSTEM FOR ASSISTING WITH THE FASTENING OF A SURGICAL IMPLANT IN A PATIENT'S BONE

(30) Priorité: 08.07.2021 FR 2107389
(43) Date de publication de la demande: 15.05.2024
(73) Titulaire: Amplitude, 26000 Valence (FR)
(72) Inventeur: THIBAUT, Sylvain, 26320 Saint Marcel Les Valences (FR); LERAT, Bernard, 26000 Valence (FR); PINET, Marylene, 26120 Montelier (FR)
(74) Mandataire: Germain Maureau
(86) Numéro de dépôt international: PCT/FR2022/051339
(87) Numéro de publication internationale: WO 2023/281204

(56) Documents cités:
- EP-A1- 3 415 110
- WO-A1-2012/109760
- WO-A1-2019/141704
- WO-A2-03/043485
- WO-A2-2007/133168
- DE-A1- 102009 025 248
- DE-A1- 102010 041 914
- DE-U1- 20 309 930
- US-A1- 2010 274 121
- US-A1- 2021 038 178

## Description

### [Domaine technique]

L'invention concerne le domaine de la fixation d'un implant chirurgical et plus particulièrement un ensemble comprenant un implant chirurgical et un système d'assistance à la fixation de l'implant dans un os d'un patient.

### [Etat de la technique]

Certaines opérations chirurgicales, notamment dans la chirurgie orthopédique, nécessitent une insertion d'un implant dans un os puis un verrouillage de cet implant dans l'os à l'aide d'au moins une clavette ou équivalent. Plus précisément, la chirurgie consiste à introduire l'implant dans l'os suivant une première direction et à verrouiller ledit implant selon une deuxième direction différente de la première direction, de sorte que le verrouillage est réalisé au travers de l'os sans que le chirurgien puisse visuellement voir l'implant.

En prévision du verrouillage, l'implant comprend, sur une partie insérée dans l'os, au moins un orifice de fixation, et préférentiellement au moins deux orifices de fixation.

Comme décrit précédemment, l'étape de verrouillage est réalisée après l'insertion de l'implant dans l'os. Ainsi, le chirurgien débutant l'étape de verrouillage ne voit plus l'implant et donc ne voit plus l'orifice de fixation.

L'étape de verrouillage consiste à réaliser, au moyen d'un outil de perçage, un orifice dans l'os, par la suite appelés orifice d'attache, en regard de l'orifice de fixation de l'implant, puis à insérer une pièce de verrouillage qui peut être une vis, une clavette ou un clou dans l'orifice d'attache et l'orifice de fixation. L'étape de verrouillage est réalisée autant de fois qu'il y a d'orifice de fixation. Il est évident que le perçage de l'os permettant de réaliser l'orifice d'attache doit être précis afin que l'orifice d'attache présente un diamètre proche de celui de l'orifice de fixation de l'implant et proche de la pièce de verrouillage.

Il existe des solutions permettant de faciliter la réalisation de l'orifice d'attache en regard de l'orifice de fixation.

Par exemple, une solution connue du document DE102010041914 consiste à utiliser un cadre de localisation dont une première extrémité est fixée à l'implant et une seconde extrémité porte un guide de perçage dont un axe d'élongation coïncide avec l'orifice de fixation.

Cependant, le cadre de localisation est encombrant, couteux à produire et nécessite un assemblage préalable de plusieurs pièces pouvant entrainer des erreurs de positionnement. En outre, il arrive que le cadre de localisation soit déformé par inadvertance ce qui empêche la réalisation de l'orifice d'attache en regard de l'orifice de fixation.

Il est également connu du document WO03043485 une solution utilisant des clichés par rayons x permettant de déterminer la localisation de l'orifice de fixation et de l'outil de perçage. Dans cette solution, un cliché par rayon x est réalisé tant que l'outil de perçage n'est pas dans une position idéale pour réaliser l'orifice d'attache en regard de l'orifice de fixation.

Cette solution entraine une irradiation du patient ainsi que du chirurgien et est longue à mettre en œuvre.

L'état de la technique peut également être illustré par les documents WO2007/133168 et US2010/0274121 qui proposent des systèmes d'assistance présentant un premier capteur inséré à l'intérieur de l'implant et un second capteur monté sur un guide de perçage, où ces capteurs sont des capteurs actifs, par exemple magnétiques, qui se détectent mutuellement et qui sont reliés, généralement par voie filaire, à une unité de traitement qui réceptionne les données de mesure de ces deux capteurs. Ces solutions nécessitent donc que le chirurgien déplace « à l'aveugle » le guide de perçage au-dessus de l'os jusqu'à ce que les données de mesure des deux capteurs renseignent un alignement entre le guide de perçage et l'orifice de fixation dans l'implant. Une telle procédure est donc peu pratique, et également peu fiable lorsqu'il s'agit d'aligner le guide de perçage successivement avec deux orifices de fixation de l'implant, car les données de mesure des capteurs ont une sensibilité souvent insuffisante pour distinguer deux orifices de fixation proches de 1 à 3 centimètres.

C'est pourquoi il existe encore un besoin pour un moyen technique fiable permettant le perçage de l'os en regard de l'orifice de fixation de l'implant.

### [Résumé de l'invention]

L'invention a pour objet de fournir un moyen technique résolvant au moins en partie les inconvénients décrits ci-dessus.

Un autre objet de l'invention est de fournir un moyen technique pour permettre au chirurgien de gagner du temps de façon à réduire le niveau de stress qu'il subit lors de l'opération chirurgicale, et de façon à réduire le risque d'infection pour le patient.

Un autre objet de l'invention est de permettre au chirurgien d'améliorer le positionnement de l'outil de perçage par rapport à l'orifice de fixation, et ainsi in fine d'offrir au patient une fixation d'implant optimale.

Un autre objet de l'invention est de proposer un moyen technique facile d'utilisation de sorte à diminuer les risques de mauvais montage lors de l'utilisation par l'équipe chirurgicale.

Un autre objet de l'invention est de diminuer les coûts de fabrication d'un implant en diminuant la précision du positionnement de l'orifice de fixation sur l'implant.

Un ou plusieurs de ces objets sont remplis par l'ensemble selon la revendication indépendante.

Les revendications dépendantes fournissent en outre des solutions à cet objet et/ou d'autres avantages.

L'invention a pour objet un ensemble comprenant un implant chirurgical et un système d'assistance pour fixer l'implant à un os au moyen d'une pièce de verrouillage insérée dans un orifice d'attache de l'os, lequel est configuré pour être positionné en regard d'un orifice de fixation de l'implant, le système d'assistance comprenant :
- un premier dispositif configuré pour être couplé mécaniquement à l'implant et comprenant un premier élément de positionnement configuré pour être détecté par un module de localisation;
- un second dispositif comprenant un second élément de positionnement configuré pour être détecté par le module de localisation et un guide de perçage configuré pour indiquer un axe de perçage ;
- un module de localisation qui comprend au moins un capteur utilisant une technologie de type détecteur d'image ou détecteur infrarouge, ledit module de localisation étant configuré pour déterminer un positionnement du premier élément de positionnement et du second élément de positionnement dans un espace tridimensionnel d'un référentiel associé au module de localisation ;
- un module de traitement relié au module de localisation pour recevoir le positionnement du premier élément de positionnement et du second positionnement ;
- un module afficheur relié au module de traitement et apte à représenter des éléments visuels indiquant un positionnement relatif de l'orifice de fixation par rapport au guide de perçage déterminé par ledit module de traitement ;

le système d'assistance étant remarquable en ce que le second dispositif comprend également au moins un élément de calibration configuré pour coopérer avec l'orifice de fixation de l'implant lors d'une étape de calibration, avant introduction de l'implant dans l'os,
et en ce que le module de traitement est conformé pour :
   - lors de l'étape de calibration, déterminer un positionnement de l'orifice de fixation dans un référentiel du premier dispositif à partir du positionnement du premier élément de positionnement et du second élément de positionnement et de côtes mécaniques référençant la position de l'au moins un élément de calibration du second dispositif dans un référentiel du second dispositif ; et pour
   - une fois l'implant introduit dans l'os, déterminer le positionnement relatif de l'orifice de fixation par rapport au guide de perçage à partir du positionnement du premier élément de positionnement et du second élément de positionnement, du positionnement de l'orifice de fixation dans le référentiel du premier dispositif, et de côtes mécaniques précisant la position du guide de perçage dans le référentiel du second dispositif.

Ainsi, le système d'assistance permet de positionner le guide de perçage en regard de l'orifice de fixation de l'implant sans qu'un lien mécanique, physique ou filaire existe entre le premier dispositif et le second dispositif, mais aussi entre le premier dispositif et le module de traitement et entre le second dispositif et le module de traitement. C'est au moyen du module de localisation, du module afficheur et du module de traitement qui sont à distance du premier dispositif et du second dispositif, que le chirurgien intervenant dans la fixation de l'implant connait précisément l'emplacement de l'orifice de fixation par rapport au guide de perçage.

Comme indiqué plus haut, le module de localisation comprend un capteur reposant sur une technologie de détection d'image ou de détection infrarouge, afin de détecter les positionnements du premier élément de positionnement du premier dispositif et du second élément de positionnement du second dispositif. Selon différentes variantes de réalisation, ce capteur correspond par exemple à un appareil photographique ou une caméra, pouvant fonctionner dans le visible ou dans l'infrarouge (voire dans une autre gamme de longueur d'onde).

Il est donc bien clair que le premier élément de positionnement et le second élément de positionnement forment des éléments passifs, car détectés par imagerie (en visible, en infrarouge ou autre), et ne nécessitent pas de connexion entre eux et avec les modules de localisation et de traitement.

Il est à noter que le module de traitement est en liaison avec une mémoire stockant les côtes mécaniques référençant la position de l'au moins un élément de calibration du second dispositif dans un référentiel du second dispositif, et les côtes mécaniques précisant la position du guide de perçage dans le référentiel du second dispositif.

Il est à noter que le module de traitement est conformé pour transmettre le positionnement relatif de l'orifice de fixation par rapport au guide de perçage au module afficheur afin que ce dernier l'affiche et le représente au moyen des éléments visuels.

Lors d'une utilisation du système d'assistance par le chirurgien, celui-ci débute par une étape de couplage mécanique lors de laquelle le chirurgien assemble le premier dispositif à l'implant. Le premier dispositif est donc solidaire de l'implant. Il n'y a pas de mouvement relatif de l'un par rapport à l'autre.

Ensuite, le chirurgien réalise une étape de calibration lors de laquelle le second dispositif est approché de l'implant de sorte que l'au moins un élément de calibration du second dispositif coïncide et coopère avec l'orifice de fixation de l'implant. Cette étape de calibration est rendue possible par l'au moins un élément de calibration que présente le second dispositif. Pour la mise en œuvre de cette étape de calibration, le chirurgien fait donc coopérer l'au moins un élément de calibration du second dispositif avec l'orifice de fixation de l'implant en le faisant entrer à l'intérieur. Lors de l'étape de calibration, le module de localisation détecte à la fois le premier élément de positionnement (qui est monté sur l'implant) et le second élément de positionnement (qui est monté dans le ou les orifices de fixation de l'implant), afin de pouvoir déterminer, dans un référentiel tridimensionnel qui lui est propre, un positionnement du premier élément de positionnement du premier dispositif et également le positionnement du second dispositif, qui est lui aussi exprimé dans le référentiel associé au module de localisation. Lors de l'étape de calibration, les positionnements du premier et du second dispositifs sont transmis au module de traitement qui détermine au moyen de côtes mécaniques référençant la position de l'au moins un élément de calibration du second dispositif dans un référentiel du second dispositif, le positionnement de l'orifice de fixation dans un référentiel du premier dispositif. En outre, comme le guide de perçage est fixe par rapport à l'au moins un élément de calibration, les côtes mécaniques référencent également une position du guide de perçage du second dispositif dans un référentiel du second dispositif.

Le chirurgien introduit ensuite l'implant toujours équipé du premier dispositif dans l'os du patient lors d'une étape d'introduction, faisant que ce premier dispositif est fixé à l'implant de sorte que lorsque l'implant est introduit dans l'os, celui-ci reste détectable par le module de localisation qui peut déterminer son positionnement dans l'espace tridimensionnel associé à son référentiel au moyen du premier élément de positionnement. L'implant, et donc l'orifice de fixation, n'est alors plus visible par le chirurgien. Seul le premier élément de positionnement du premier dispositif reste détectable (car visible) par le module de localisation. Le positionnement de ce premier élément de positionnement est exprimé dans le référentiel associé au module de localisation. Le chirurgien peut ensuite réaliser une étape de placement du second dispositif suivant les éléments visuels du module afficheur.

Autrement dit, le chirurgien approche le second dispositif de l'os de sorte que le guide de perçage indique l'axe de perçage de l'os. Le module de localisation détermine à nouveau alors le positionnement du premier dispositif et du second dispositif dans le référentiel associé au module de localisation. Ces informations sont transmises au module de traitement qui, à l'aide du positionnement du premier et du second dispositifs dans le référentiel associé au module de localisation, du positionnement de l'orifice de fixation de l'implant dans le référentiel du premier dispositif (ainsi que déterminé au préalable lors de l'étape de calibration) et des côtes mécaniques précisant la position du guide de perçage dans le référentiel du second dispositif, en déduit le positionnement relatif de l'orifice de fixation par rapport au guide de perçage. Autrement dit, le module de traitement exprime le positionnement relatif de l'orifice de fixation par rapport au guide de perçage.

Ainsi, durant cette étape de placement, le module afficheur indique alors, au moyen d'éléments visuels, le positionnement relatif de l'orifice de fixation par rapport au guide de perçage. Le chirurgien, par la consultation du module afficheur, déplace le guide de perçage de sorte à le positionner en regard de l'orifice de fixation.

Lorsque la position du guide de perçage est satisfaisante pour le chirurgien, il effectue une étape de perçage de l'os du patient de sorte à créer l'orifice d'attache en regard de l'orifice de fixation. Pour cela il perce l'os en suivant l'axe indiqué par le guide de perçage.

Enfin, le chirurgien réalise une étape d'insertion de la pièce de verrouillage dans l'orifice d'attache et dans l'orifice de fixation. L'implant est alors fixé solidairement à l'os.

Le chirurgien recommence les étapes de placement du second dispositif, et de perçage de l'os aussi souvent qu'il y a d'orifice de fixation sur l'implant.

Après fixation de l'implant, le premier dispositif est séparé de l'implant.

Le système d'assistance selon l'invention peut être alors éventuellement nettoyé en vue d'une nouvelle utilisation, s'il n'est pas à usage unique.

Ainsi, avec le système d'assistance selon l'invention, le chirurgien gagne du temps dans la mise en œuvre du système d'assistance et dans le positionnement de l'outil de perçage de sorte à créer un orifice d'attache en regard de l'orifice de fixation.

En outre, l'au moins un élément de calibration, le module afficheur, le module de traitement et le module de localisation ainsi que le premier dispositif et le second dispositif rendent le positionnement de l'outil de perçage par rapport à l'orifice de fixation particulièrement précis. En effet, l'absence de lien mécanique ou physique entre le premier dispositif et le second dispositif (et aussi entre ces deux dispositifs et le module de localisation et le module de traitement) évite une déformation dudit lien qui entrainerait un mauvais positionnement, et évite aussi une gêne pour le chirurgien.

Le système d'assistance selon l'invention est un moyen technique facile d'utilisation en ce que le nombre de pièce du module est limité. Le chirurgien ne manipule que le premier dispositif et le second dispositif. Il n'y a donc pas de risques de mauvais montage lors de l'utilisation par l'équipe chirurgicale.

Enfin, l'utilisation du système d'assistance selon l'invention permet de diminuer les coûts de fabrication de l'implant. En effet, l'au moins un élément de calibration permet de connaitre exactement le positionnement de l'au moins un orifice de fixation sur l'implant. Il n'est donc pas utile que, lors de la fabrication de l'implant, l'orifice de fixation soit réalisé avec une grande précision de positionnement.

Selon un mode de réalisation, le guide de perçage est un tube.

Selon un mode de réalisation, l'implant comprend une pluralité d'orifice de fixation.

L'invention peut également présenter une ou plusieurs des caractéristiques suivantes prises seules ou en combinaison.

Selon un mode de réalisation, le guide de perçage comprend au moins une dent de stabilisation configurée pour stabiliser le guide de perçage sur l'os.

Selon un mode de réalisation, le guide de perçage comprend une pluralité de dents de stabilisation.

Les dents de stabilisation sont positionnées à une extrémité du guide de perçage destinée à être en regard de l'os du patient. Les dents de stabilisation facilitent le maintien du guide de perçage dans la position souhaitée par le chirurgien.

Selon un mode de réalisation, le second dispositif présente une partie de maintien configurée pour permettre son maintien lors de l'utilisation du système d'assistance.

Selon un mode de réalisation, la partie de maintien est confondue avec le guide de perçage. Selon un mode de réalisation, un diamètre intérieur du guide de perçage est choisi pour faciliter l'introduction d'un outil de perçage à l'intérieur des orifices de fixation de l'implant.

Selon un mode de réalisation, l'au moins un élément de calibration est une élongation configurée pour être introduite dans l'orifice de fixation.

Selon un mode de réalisation, l'élongation est choisie parmi un plot et une goupille.

Selon un mode de réalisation, l'élongation de l'au moins un élément de calibration s'étend suivant un axe sensiblement parallèle à l'axe de perçage du guide de perçage.

Selon un mode de réalisation, l'élongation de l'au moins un élément de calibration a un diamètre inférieur mais proche de celui de l'orifice de fixation de sorte à pouvoir être introduite dans l'orifice de fixation.

Selon une possibilité, l'au moins un élément de calibration comprend au moins deux éléments de calibration s'étendant parallèlement, ce qui est avantageux pour avoir un montage stable du second dispositif dans les orifices de fixation de l'implant lors de l'étape de calibration.

Selon un mode de réalisation, le premier élément de positionnement et/ou le second élément de positionnement sont choisis parmi un capteur et une géométrie, ladite géométrie état choisie parmi : un QR code, une image fixe, un corps rigide comprenant au moins un marqueur réfléchissant ou au moins trois élongations positionnées suivant trois direction différentes. Selon un mode de réalisation, le corps rigide comprend au moins trois sphères réfléchissantes. Lorsque le module de localisation comprend un capteur basé sur une ou plusieurs caméras infrarouges, le premier élément de positionnement et/ou le second élément de positionnement ont des marqueurs réfléchissants qui sont fixés solidairement. Les marqueurs peuvent être des sphères réfléchissant les rayons infrarouges.

Le corps rigide peut comprendre par exemple au moins trois élongations positionnées suivant trois directions différentes, le positionnement relatif des élongations entre-elles étant fixe.

Les élongations permettent de déterminer un plan de référence et ainsi permettent au module de localisation et au module de traitement de déterminer la position du premier dispositif et du second dispositif dans le référentiel du module de localisation.

Un module de localisation utilisant des détecteurs infrarouges présente une précision importante.

Selon un mode de réalisation, le module de localisation utilisé a une précision inférieure à 0,5 mm RMS selon la norme ASTM F2554-18, ou 0,2 mm et préférentiellement inférieure à 0,15 mm.

Selon un mode de réalisation, chacune des au moins trois sphères réfléchissantes est positionnée respectivement à une extrémité libre de l'une des au moins trois élongations. Selon un mode de réalisation, le module de localisation est fixe dans un référentiel associé au module afficheur.

Ainsi le module de localisation et le module afficheur ont le même référentiel.

Selon un mode de réalisation, le module de localisation et le module afficheur sont solidaires. Selon un mode de réalisation, le module de traitement est solidaire du module afficheur.

Ainsi, le système d'assistance est particulièrement simple à installer.

Selon un mode de réalisation, le module afficheur est un écran, et par exemple un écran de télévision ou un écran d'ordinateur.

Selon un mode de réalisation, les éléments visuels montrent l'axe de perçage et une mire de perçage configurée pour indiquer un positionnement optimal d'un outil de perçage par rapport à l'orifice de fixation.

Selon un mode de réalisation, les éléments visuels indiquent l'orifice de fixation le plus proche du guide de perçage.

Selon un mode de réalisation, le système d'assistance utilise une technologie de réalité augmentée dans lequel le module afficheur est apte à représenter les éléments visuels, en superposition dans le champ visuel d'un utilisateur du système d'assistance.

Dans ce cas, le module afficheur est intégré à un casque, des lunettes ou des lentilles de contact qui sont portées par le chirurgien. Ainsi, le chirurgien voit directement l'os à percer et en superposition sur celui-ci les éléments visuels d'assistance au positionnement du guide de perçage.

Selon un mode de réalisation, le module afficheur comprend un dispositif de localisation, le système d'assistance comprenant un module d'appariement apte à déterminer une relation spatiale permettant de transformer des descriptions géométriques exprimées dans un référentiel associé au module de localisation, en descriptions géométriques exprimées dans un référentiel associé au dispositif de localisation.

Afin d'obtenir une bonne précision, le système d'assistance utilisant une technologie de réalité augmentée a un module afficheur dont une position par rapport au dispositif de localisation est connue. Ainsi la position du module afficheur est connue dans le référentiel du module de localisation.

Le module d'appariement est un élément détectable à la fois par le module de localisation et à la fois par le dispositif de localisation.

Selon un mode de réalisation, le module d'appariement comprend au moins un élément d'appariement choisi parmi : une image fixe, un QR code, une géométrie tridimensionnelle, au moins trois sphères réfléchissantes, un corps rigide comprenant au moins un marqueur réfléchissant ou au moins trois élongations positionnées suivant trois direction différentes.

Il est également décrit un procédé d'utilisation d'un système d'assistance selon l'invention comprenant :
- Une étape de couplage mécanique lors de laquelle le premier dispositif est fixé à l'implant ;
- Une étape de calibration lors de laquelle l'au moins un élément de calibration du second dispositif coopère avec l'orifice de fixation de l'implant ;
- Une étape d'introduction de l'implant dans un os d'un patient ;
- Une étape de placement du second dispositif suivant les éléments visuels du module afficheur ;
- Une étape de perçage de l'os de façon à créer l'orifice d'attache en regard de l'orifice de fixation ;
- Une étape d'insertion de la pièce de verrouillage dans l'orifice d'attache et dans l'orifice de fixation.

Lorsque l'implant comprend plusieurs orifices de fixation, les étapes de placement, de perçage et d'insertion sont refaites autant de fois qu'il y a d'orifice de fixation.

L'invention porte sur un ensemble comprenant :
- un implant muni d'un orifice de fixation, et
- un système d'assistance tel que décrit précédemment;
dans lequel le premier dispositif du système d'assistance est configuré pour être couplé mécaniquement à l'implant, et l'au moins un élément de calibration du second dispositif du système d'assistance est conformé pour collaborer avec l'orifice de fixation de l'implant lors de l'étape de calibration à réaliser avant une introduction de l'implant dans un os.

Autrement dit, l'élément de calibration du second dispositif est de forme complémentaire audit orifice de fixation de l'implant, pour permettre un engagement par correspondance géométrique.

### [Brève description des figures]

L'invention sera mieux comprise, grâce à la description ci-après, qui se rapporte à plusieurs modes de réalisation selon la présente invention, donnés à titre d'exemples non limitatifs et expliqués avec référence aux dessins schématiques annexés, dans lesquels :
Figure 1 est une représentation d'un premier dispositif couplé à un implant chirurgical et un second dispositif selon l'invention ;
Figure 2 est une représentation du premier dispositif vue de face ;
Figure 3 une représentation du second dispositif suivant une vue latérale ;
Figure 4 une représentation du second dispositif vue de face ;
Figure 5 une représentation du premier dispositif couplé à l'implant chirurgical et du second dispositif lors d'une étape de calibration ;
Figure 6 est une représentation d'un module d'appariement qui peut être utilisé dans l'invention ;
Figure 7 est une représentation du module de localisation, du module de traitement et du module afficheur selon un premier mode de réalisation ;
Figure 8 est une représentation des éléments visuels du module afficheur selon le premier mode de réalisation lorsqu'un guide de perçage n'est pas en regard d'un orifice de fixation ;
Figure 9 est une représentation des éléments visuels du module afficheur selon le premier mode de réalisation lorsque le guide de perçage est en regard de l'orifice de fixation ;
Figure 10 est une représentation des éléments visuels du module afficheur selon un second mode de réalisation utilisant une technologie de réalité augmentée lorsque le guide de perçage n'est pas en regard de l'orifice de fixation ;
Figure 11 est une représentation du premier dispositif couplé à un implant chirurgical et du second dispositif selon l'invention lors d'une étape de perçage ;
Figure 12 est une représentation schématique du système d'assistance et de l'implant lors de l'étape de calibration.

### [Description détaillée d'un ou plusieurs modes de réalisation de l'invention]

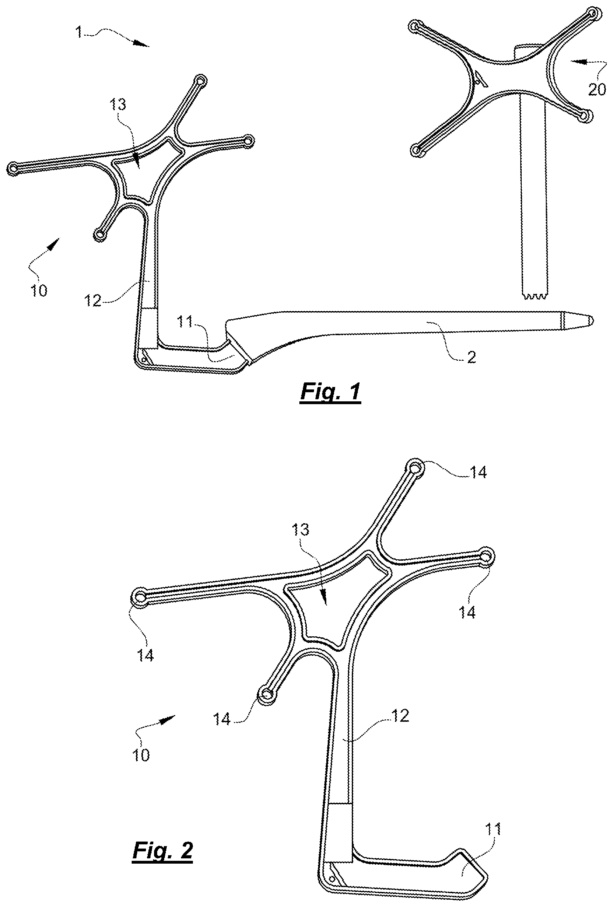

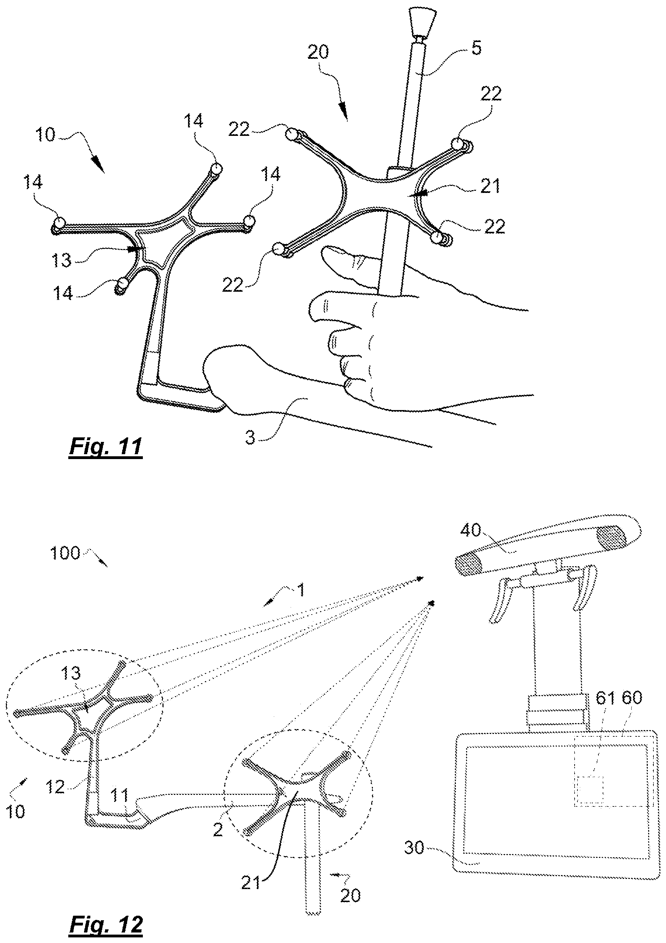

Un système d'assistance 1 selon l'invention, représenté aux figures 1 à 12, permet de fixer un implant 2 chirurgical dans un os 3 d'un patient au moyen d'une ou plusieurs pièces de verrouillage (comme par exemple une vis ou une broche), chaque pièce de verrouillage étant insérée dans un orifice d'attache de l'os 3 et dans un orifice de fixation 4 de l'implant 2 réalisé en regard de l'orifice d'attache. Dans le cas présent, l'implant 2 présente deux orifices de fixation 4 s'étendant parallèlement à un axe transversal de l'implant 2, et qui sont de même diamètre. Le système d'assistance 1 comprend un premier dispositif 10, un second dispositif 20, un module afficheur 30, un module de localisation 40 et un module de traitement 60 relié à une mémoire 61, et en référence à la Figure 12 ce système d'assistance 1 peut être associé à l'implant 2 pour former un ensemble 100.

Le premier dispositif 10 est plus particulièrement représenté en figure 2. Ce premier dispositif 10 comprend une partie de couplage 11 qui lui permet d'être assemblé à l'implant 2 de manière mécanique et amovible. Le premier dispositif 10 et l'implant 2 sont alors solidaires l'un de l'autre. Autrement dit, tout mouvement de l'un entraine un mouvement de l'autre, et une détermination d'un positionnement de l'un permet de connaitre un positionnement de l'autre.

Le premier dispositif 10 comprend au bout d'une tige 12 un premier élément de positionnement 13. Le premier élément de positionnement 13 est un corps rigide présentant quatres élongations équipées chacune à leur extrémité libre d'une sphère réfléchissante 14. Les quatre sphères réfléchissantes 14 définissent un référentiel spatial et sont configurées pour être détectées par le module de localisation 40, et plus spécifiquement par au moins une caméra infrarouge du module de localisation 40.

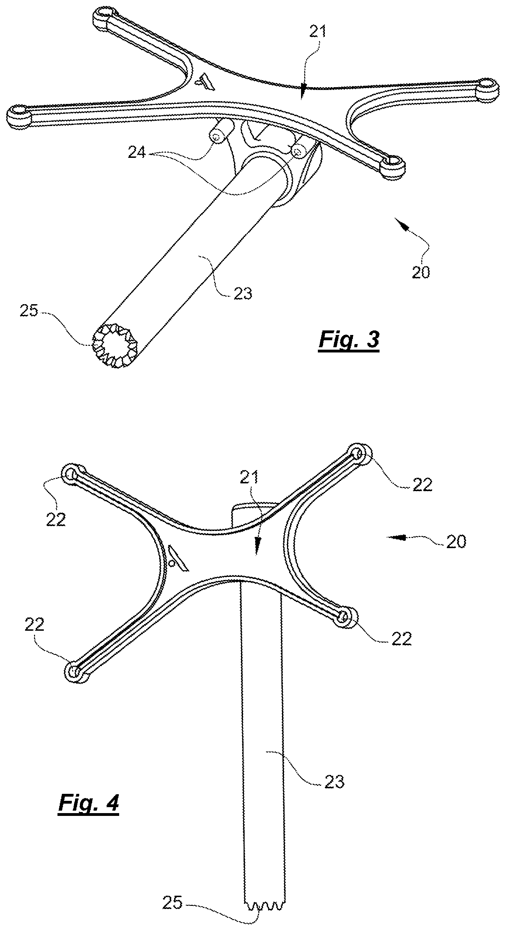

Le second dispositif 20 est plus particulièrement représenté aux figures 3 et 4. Ce second dispositif 20 comprend un second élément de positionnement 21. Le second élément de positionnement 21, à l'image du premier élément de positionnement 13, est également un corps rigide présentant quatres élongations équipées chaune à leur extrémité libre d'une sphère réfléchissante 22, lesquelles quatre sphères réfléchissantes 22 définissent un référentiel spatial et sont configurées pour être détectées par l'au moins une caméra infrarouge du module de localisation 40. Le second dispositif 20 comprend également un guide de perçage 23 et deux éléments de calibration 24. Le guide de perçage 23, les éléments de calibration 24 et le second élément de positionnement 21 sont solidaires entre eux.

Une première extrémité du guide de perçage 23 comprend des dents de stabilisation 25 qui ont une forme de dents de scie.

Le guide de perçage 23 est confondu avec une partie de maintien du second dispositif 20. Le guide de perçage 23 a donc une longueur permettant de facilement positionner une main ou une attache mécanique.

Le guide de perçage 23 a un diamètre supérieur à un outil de perçage 5 (et en particulier une mèche de perçage) utilisé pour réaliser l'orifice d'attache.

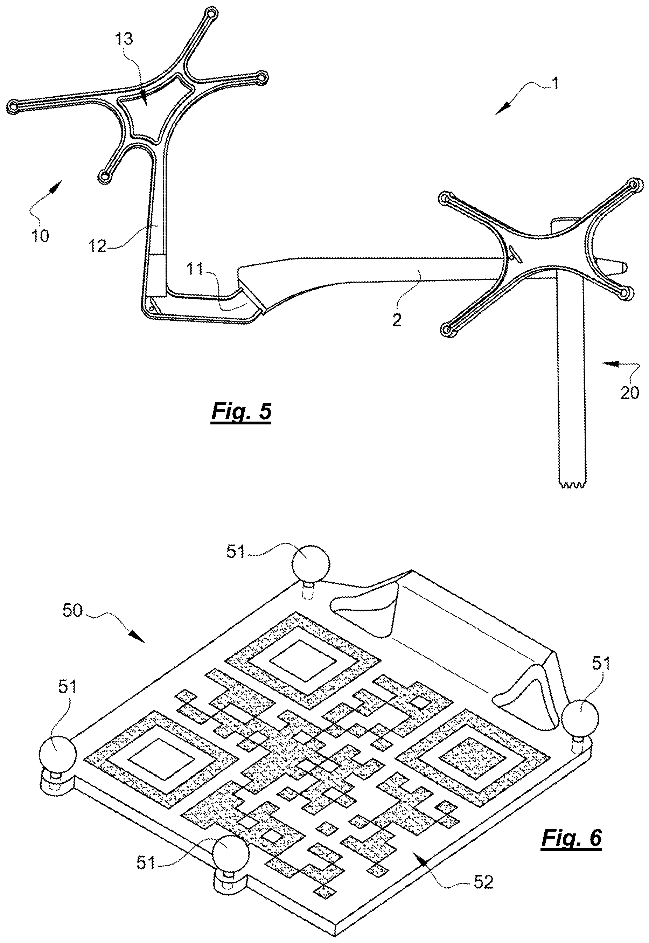

Les éléments de calibration 24 comprennent deux élongations qui sont des plots ou goupilles s'étendant suivant un axe sensiblement parallèle à l'axe de perçage du guide de perçage 23. Les élongations ont un diamètre inférieur mais proche de celui des deux orifices de fixation 4 de sorte à pouvoir être introduites dans les deux orifices de fixation 4 tel que représenté en figure 5, permettant ainsi au chirurgien de mettre en œuvre l'étape de calibration.

Le module de localisation 40 comprend par exemple deux caméras infrarouges configurées pour détecter le positionnement des sphères réfléchissantes 14, 22 équipant le premier 13 et le second 21 éléments de positionnement dans un espace tridimensionnel. Une distance entre les deux caméras infrarouges est fixe et connue.

Nous allons par la suite détailler plusieurs modes de réalisation.

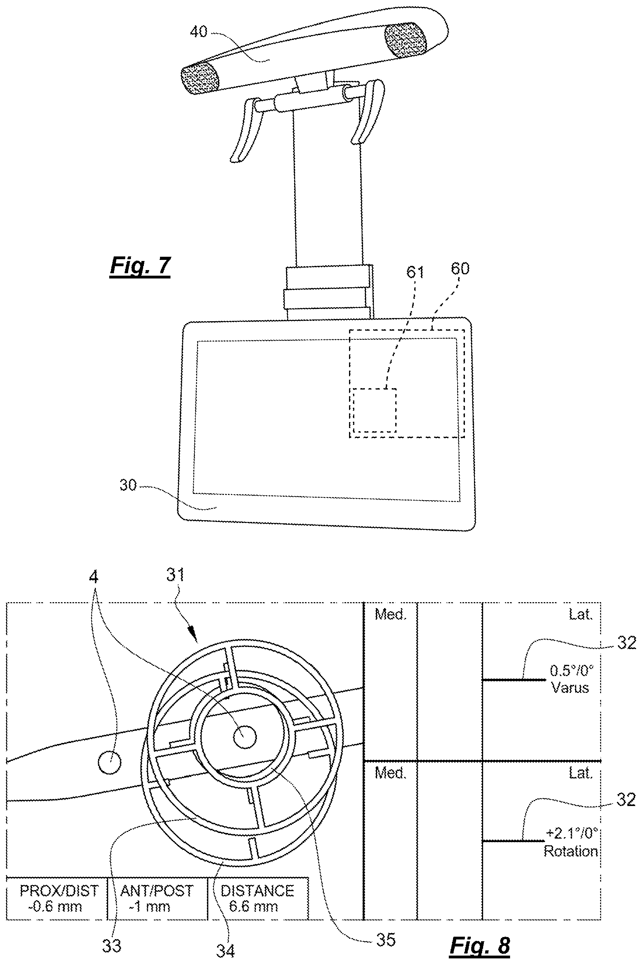

Le premier mode de réalisation est celui dans lequel le module afficheur 30 est un écran d'ordinateur tel que représenté en figure 7. Ce mode de réalisation est détaillé dans les paragraphes suivants.

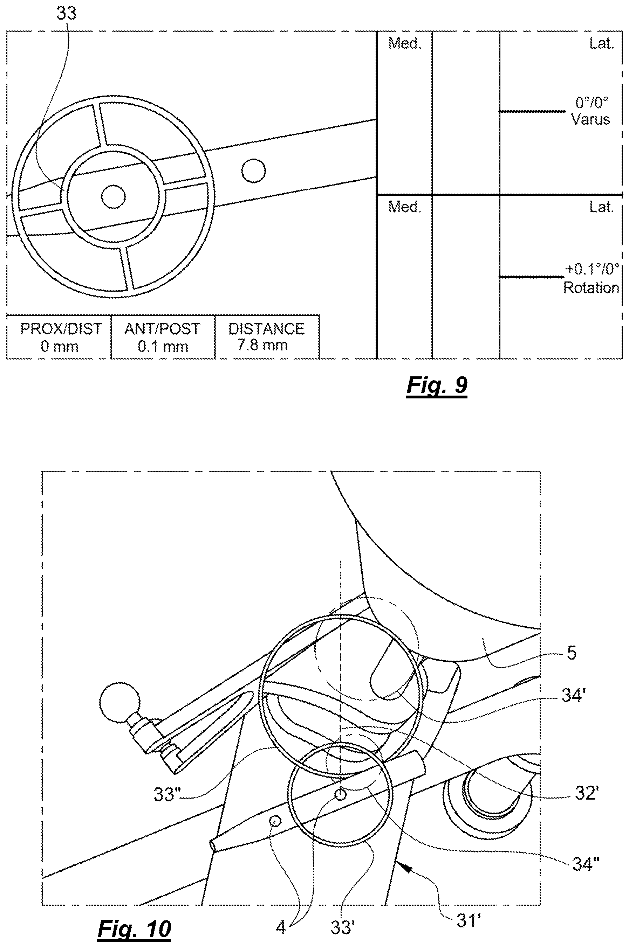

Le module afficheur 30 permet de représenter des éléments visuels 31 indiquant un positionnement relatif des orifices de fixation 4 par rapport au guide de perçage 23 sur une coupe vue de dessus, et une coupe latérale de l'implant 2. Les éléments visuels 31 correspondent à l'axe de perçage 32, une mire de perçage 33 correspondant au positionnement optimal de l'outil de perçage 5 par rapport à l'orifice de fixation 4 correspondant, mais également à deux cercles d'orientation 34, 35 qui indiquent l'axe de perçage vu de dessus, tels qu'illustrés en figures 8 et 9. Lorsque les deux cercles d'orientation 34, 35 sont confondus l'un avec l'autre et concentriques avec la mire de perçage 33, comme cela est représenté en figure 9, l'outil de perçage 5 est dans une position optimale.

Le module de traitement 60 est par exemple une carte électronique, un processeur, un contrôleur, un ordinateur. Ce module de traitement 60 transforme le positionnement du premier élément de positionnement 13 et du second élément de positionnement 21 en positionnement relatif des orifices de fixation 4 par rapport au guide de perçage 23.

Dans ce mode de réalisation, le module afficheur 30, le module de traitement 60 et le module de localisation 40 sont solidaires entre-eux.

Le deuxième mode de réalisation est celui dans lequel le module afficheur 30 est un casque de réalité augmentée et dans lequel le module de localisation 40 est intégré au casque de réalité virtuel. Il est explicité en référence à la figure 10. Ce mode de réalisation est détaillé dans les paragraphes suivants.

Le module afficheur 30 permet de représenter des éléments visuels 31' indiquant le positionnement relatif de l'orifice de fixation 4 par rapport au guide de perçage 23, en superposition dans le champ visuel d'un chirurgien utilisateur du système d'assistance. Les éléments visuels 31' correspondent à l'axe de perçage 32', à une mire de perçage 33', 33" correspondant au positionnement optimal de l'outil de perçage 5 par rapport à l'orifice de fixation 4, mais également deux cercles d'orientation 34', 34", qui indiquent l'axe de perçage, tels qu'illustrés en figure 10. Lorsque les cercles d'orientation 34', 34" sont coaxiaux avec les mires de perçages 33', 33" l'outil de perçage 5 est dans une position optimale.

Dans ce mode de réalisation, le module afficheur 30, le module de traitement 60 et le module de localisation 40 peuvent être solidaires du casque de réalité augmenté.

Le module de traitement 60 est intégré au casque de réalité augmentée et transforme le positionnement du premier élément de positionnement 13 et du second élément de positionnement 21 en positionnement relatif des orifices de fixation 4 par rapport au guide de perçage 23.

Le module afficheur 30 permet, comme dans le deuxième mode de réalisation, de représenter des éléments visuels 31' indiquant un positionnement relatif de l'orifice de fixation 4 par rapport au guide de perçage 23, en superposition dans le champ visuel d'un chirurgien utilisateur du système d'assistance. Les éléments visuels 31' correspondent à l'axe de perçage 32' et à la mire de perçage 33', 33" correspondant au positionnement optimal de l'outil de perçage 5 par rapport à l'orifice de fixation 4 mais également deux cercles d'orientation 34', 34" qui indiquent l'axe de perçage, tels qu'illustrés en figure 10. Lorsque les cercles d'orientation 34', 34" sont coaxiaux avec les mires de perçage 33', 33" l'outil de perçage 5 est dans une position optimale.

Le casque de réalité augmentée comporte un dispositif de localisation (qui comprend au moins une caméra), définissant un référentiel associé au dispositif de localisation, par la suite appelé MLCRA, permettant au casque de réalité augmentée de déterminer son propre positionnement dans un espace tridimensionnel. Son positionnement est exprimé dans le référentiel MLCRA. Le casque de réalité augmentée comporte donc une caméra définissant un référentiel associé à la caméra, par la suite appelé MLC, permettant de reconnaître des formes géométriques ou des images imprimées, et de déterminer leur positionnement dans le référentiel du dispositif de localisation MLCRA via une transformation connue et fixe du système MLC2MLCRA.

Le casque de réalité augmentée comporte un module de traitement 60 (comme une carte électronique ou un processeur) qui permet au module afficheur 30 d'afficher des représentations graphiques en surimpression du réel dès lors qu'elles sont exprimées dans le référentiel MLCRA de sorte à correspondre à leur positionnement réel.

Le troisième mode de réalisation est celui dans lequel le module afficheur 30 est un casque de réalité augmentée et dans lequel le module de localisation 40 (qui comprend une ou plusieurs caméras) n'est pas intégré au casque de réalité virtuel. Le module afficheur 30 n'est ainsi pas solidaire du module de localisation 40 qui détermine donc un positionnement des premier 10 et second 20 dispositifs dans un référentiel associé au module de localisation 40, par la suite référentiel MLE. Ainsi il existe un mouvement relatif entre le module afficheur (ou le casque de réalité augmentée) et le module de localisation 40. Ce mode de réalisation est détaillé dans les paragraphes suivants, en référence à la figure 10.

Dans ce troisième mode de réalisation, le système d'assistance 1 comprend également un module d'appariement 50 qui permet au module de traitement 60 de définir une relation spatiale MLE2MLCRA entre le module de localisation 40 et le dispositif de localisation (à savoir la caméra porté par le casque de réalité virtuel). Cette relation spatiale MLE2MLCRA permet de transformer des descriptions géométriques exprimées dans le référentiel MLE, en descriptions géométriques exprimées dans le référentiel MLCRA. Autrement dit, le module d'appariement 50 permet de lier les modules de sorte que le module de traitement 60, potentiellement intégré au casque de réalité augmentée ou au module afficheur 30, transforme le positionnement du premier dispositif 10 et du second dispositif 20 tous deux exprimés dans le référentiel MLE en positionnement relatif de l'orifice de fixation 4 par rapport au guide de perçage 23 et les exprime dans un référentiel associé au module afficheur 30 via le référentiel du dispositif de localisation MLCRA. Cette transformation MLE2MLCRA restera valable tant que le module de localisation 40 et le dispositif de localisation ne seront pas déplacés dans l'espace.

Le module d'appariement 50, représenté en figure 6, est un élément détectable à la fois par le module de localisation 40 et par le dispositif de localisation du casque de réalité virtuel. Le module d'appariement 50 comprend par exemple des sphères réfléchissantes 51 détectables par la ou les caméras infrarouges du module de localisation 40 et un QR code 52 détectable par la caméra du dispositif de localisation du casque de réalité augmentée. Le QR Code 52 et les sphères réfléchissantes 51 étant solidaires, le module de traitement 60 connait une relation spatiale fixe SPHERE2QR entre les sphères réfléchissantes 51 et le QR code 52.

Lors d'une étape de liaison des modules, le module d'appariement 50 est positionné à un endroit fixe puis il est détecté simultanément, ou séquentiellement, par le module de localisation 40 et par le dispositif de localisation ou la caméra du casque de réalité augmentée puis le module de traitement 60 détermine la relation spatiale MLE2MLCRA permettant de transformer les descriptions géométriques exprimées dans le référentiel MLE, en descriptions géométriques exprimées dans le référentiel MLCRA. Plus précisémment, la détection du module d'appariement 50 par le module de localisation 40 permet de déterminer la relation MLE2SPHERE. La détection du module d'appariement 50 par le dispositif de localisation, ou la caméra du casque de réalité augmentée, permet de déterminer la relation QR2MLC dans le référentiel du premier dispositif 10. Ainsi, la relation spatiale MLE2MLCRA déterminée par le module de traitement correspond aux transformations MLE2SPHERE x SPERE2QR x QR2MLC x MLC2MLCRA.

Dans le cas de l'utilisation du système d'assistance 1 selon le troisième mode de réalisation, le chirurgien installe le casque de réalité augmentée sur sa tête et réalise l'étape de liaison des modules telle que décrite ci-dessus.

Ensuite le chirurgien effectue une étape de couplage mécanique lors de laquelle il assemble le premier dispositif 10 à l'implant 2.

Puis, le chirurgien réalise une étape de calibration lors de laquelle le second dispositif 20 est approché de l'implant 2 de sorte que les éléments de calibration 24 du second dispositif 20 s'insèrent simultanément ou non dans les orifices de fixation 4 de l'implant 2 ; les éléments de calibration 24 venant en engagement par correspondance géométrique dans les orifices de fixation 4 de l'implant 2.

Lors de l'étape de calibration, les positionnements du premier 10 et du second 20 dispositif sont déterminés/détectés par le module de localisation 40. Ces informations sont transmises au module de traitement 60 qui détermine ainsi de façon précise, au moyen des positionnements du premier 10 et du second 20 dispositif et de côtes mécaniques référençant la position des éléments de calibration 24 du second dispositif 20 dans un référentiel du second dispositif 20, un positionnement des orifices de fixation 4 dans le référentiel du premier dispositif 10. A la fin de cette étape de calibration, le chirurgien désaccouple le second dispositif 20 de l'implant 2.

Le chirurgien introduit ensuite l'implant 2 dans l'os 3 lors d'une étape d'introduction de l'implant 2. L'implant 2, et donc les orifices de fixation 4, ne sont alors plus visibles par le chirurgien. Seul le premier élément de positionnement 13 est détectable par le module de localisation 40.

Le chirurgien réalise une étape de placement du second dispositif 20, telle qu'illustrée en figure 11, en approchant le second dispositif 20 de l'os 3. Le module de localisation 40 détermine à nouveau le positionnement du premier dispositif 10 et du second dispositif 20 dans le référentiel associé au module de localisation 40. Ces informations sont transmises au module de traitement 60 qui, à l'aide du positionnement du premier 10 et du second 20 dispositifs dans le référentiel associé au module de localisation, du positionnement des orifices de fixation 4 de l'implant 2 dans le référentiel du premier dispositif 10 et de côtes mécaniques précisant la position du guide de perçage 23 dans le référentiel du second dispositif 20, en déduit le positionnement relatif de l'orifice de fixation 4 par rapport au guide de perçage 23.

Il est à noter que les côtes mécaniques référençant la position des éléments de calibration 24 et du guide de perçage 23 du second dispositif 20 dans un référentiel du second dispositif 20, sont stockées dans la mémoire 61 reliée au module de traitement 60.

Le module de traitement 60 est en communication avec le module afficheur 30 afin de lui transmettre le positionnement relatif de l'orifice de fixation 4 par rapport au guide de perçage 23, afin que ce module afficheur 30 affiche et ce positionnement relatif et le représente au moyen des éléments visuels 31, 31'.

Les éléments visuels 31' du module afficheur indique alors le positionnement relatif du ou des orifices de fixation 4 par rapport au guide de perçage 23. Le chirurgien, par la consultation du module afficheur 30, déplace le guide de perçage 23 de sorte à le positionner en regard de l'orifice de fixation 4 concerné.

Lorsque la position du guide de perçage 23 est validée, le chirurgien effectue une étape de perçage de l'os 3 du patient de sorte à créer l'orifice d'attache dans l'os 3 en regard de l'orifice de fixation 4 correspondant. Pour cela il perce l'os 3 en introduisant l'outil de perçage 5 dans le guide de perçage 23.

Enfin, le chirurgien réalise une étape d'insertion d'une pièce de verrouillage dans l'orifice d'attache et dans l'orifice de fixation 4. Les opérations de perçage sont répétées en fonction du nombre d'orifices de fixation 4 et donc du nombre de fixations à mettre en place. L'implant 2 est alors fixé solidairement à l'os 3, le premier dispositif 10 est alors séparé de l'implant 2.

Bien entendu, l'invention n'est pas limitée aux modes de réalisation décrits et représentés aux figures annexées. Des modifications restent possibles, notamment du point de vue de la constitution des divers éléments ou par substitution d'équivalents techniques, sans sortir pour autant du domaine de protection de l'invention.

## Revendications

1. Ensemble comprenant :
- un implant (2) muni d'au moins un orifice de fixation (4), et
- un système d'assistance (1) pour fixer ledit implant (2) chirurgical à un os (3) au moyen d'une pièce de verrouillage insérée dans un orifice d'attache de l'os, lequel est configuré pour être positionné en regard de l'orifice de fixation (4) de l'implant (2),
le système d'assistance (1) comprenant :
- un premier dispositif (10) configuré pour être couplé mécaniquement à l'implant (2) et comprenant un premier élément de positionnement (13) configuré pour être détecté par un module de localisation (40);
- un second dispositif (20) comprenant un second élément de positionnement (21) configuré pour être détecté par le module de localisation (40) et un guide de perçage (23) configuré pour indiquer un axe de perçage (32, 32') ;
- le module de localisation (40) qui comprend au moins un capteur utilisant une technologie de type détecteur d'image ou détecteur infrarouge, ledit module de localisation (40) étant configuré pour déterminer un positionnement du premier élément de positionnement (13) et du second élément de positionnement (21) dans un espace tridimensionnel d'un référentiel associé au module de localisation (40) ;
- un module de traitement (60) relié au module de localisation (40) pour recevoir le positionnement du premier élément de positionnement (13) et du second élément de positionnement (21) ;
- un module afficheur (30) relié au module de traitement (60) et apte à représenter des éléments visuels (31, 31') indiquant un positionnement relatif de l'orifice de fixation (4) par rapport au guide de perçage (23) déterminé par le module de traitement (60) ;
l'ensemble étant **caractérisé en ce que** le second dispositif (20) comprend également au moins un élément de calibration (24) configuré pour coopérer avec l'orifice de fixation (4) de l'implant (2) lors d'une étape de calibration, avant introduction de l'implant (2) dans l'os (3),
**et en ce que** le module de traitement (60) est conformé pour :
- lors de l'étape de calibration, déterminer un positionnement de l'orifice de fixation (4) dans un référentiel du premier dispositif (10) à partir du positionnement du premier élément de positionnement (13) et du second élément de positionnement (21) et de côtes mécaniques référençant la position de l'au moins un élément de calibration (24) du second dispositif (20) dans un référentiel du second dispositif (20) ; et pour
- une fois l'implant (2) introduit dans l'os (3), déterminer le positionnement relatif de l'orifice de fixation (4) par rapport au guide de perçage (23) à partir du positionnement du premier élément de positionnement (13) et du second élément de positionnement (21), du positionnement de l'orifice de fixation (4) dans le référentiel du premier dispositif (10), et de côtes mécaniques précisant la position du guide de perçage (23) dans le référentiel du second dispositif (20).

2. Ensemble selon la revendication 1, dans lequel le guide de perçage (23) comprend au moins une dent de stabilisation (25) configurée pour stabiliser le guide de perçage (23) sur l'os (3).

3. Ensemble selon l'une quelconque des revendications précédentes, dans lequel l'au moins un élément de calibration (24) est une élongation configurée pour être introduite dans l'orifice de fixation (4).

4. Ensemble selon la revendication 3, dans lequel l'élongation est choisie parmi un plot et une goupille.

5. Ensemble selon la revendication 3 ou 4, dans lequel l'élongation de l'au moins un élément de calibration (24) s'étend suivant un axe sensiblement parallèle à l'axe de perçage (32, 32') du guide de perçage (23).

6. Ensemble selon l'une quelconque des revendications précédentes, dans lequel l'au moins un élément de calibration (24) comprend au moins deux éléments de calibration (24) s'étendant parallèlement.

7. Ensemble selon l'une quelconque des revendications précédentes, dans lequel le premier élément de positionnement (13) et/ou le second élément de positionnement (21) sont choisis parmi un capteur et une géométrie, ladite géométrie étant choisie parmi : un QR code, une image fixe, un corps rigide comprenant au moins un marqueur réfléchissant ou au moins trois élongations positionnées suivant trois directions différentes.

8. Ensemble selon l'une quelconque des revendications précédentes, dans lequel le module de localisation est fixe dans un référentiel associé au module afficheur.

9. Ensemble selon l'une quelconque des revendications précédentes, dans lequel les éléments visuels (31, 31') montrent l'axe de perçage (32, 32') et une mire de perçage (33, 33', 33") configurée pour indiquer un positionnement optimal d'un outil de perçage (5) par rapport à l'orifice de fixation (4).

10. Ensemble selon l'une quelconque des revendications précédentes, utilisant une technologie de réalité augmentée dans lequel le module afficheur est apte à représenter les éléments visuels (31'), en superposition dans le champ visuel d'un utilisateur du système d'assistance.

11. Ensemble selon la revendication 10, dans lequel le module afficheur comprend un dispositif de localisation, le système d'assistance comprenant un module d'appariement (50) apte à déterminer une relation spatiale permettant de transformer des descriptions géométriques exprimées dans un référentiel associé au module de localisation, en descriptions géométriques exprimées dans un référentiel associé au dispositif de localisation.

## Patentansprüche

1. Baugruppe, umfassend:
- ein Implantat (2), das mit mindestens einer Befestigungsöffnung (4) versehen ist, und
- ein Hilfssystem (1) zum Befestigen des chirurgischen Implantats (2) an einem Knochen (3) mithilfe eines Verriegelungsteils, der in eine Verankerungsöffnung des Knochens eingeführt wird und so konfiguriert ist, dass es gegenüber der Befestigungsöffnung (4) des Implantats (2) positioniert werden kann,
wobei das Hilfssystsem (1) Folgendes umfasst:
- eine erste Vorrichtung (10), die so konfiguriert ist, dass sie mechanisch mit dem Implantat (2) gekoppelt werden kann, und ein erstes Positionierungselement (13) umfasst, das so konfiguriert ist, dass es von einem Ortungsmodul (40) erfasst werden kann;
- eine zweite Vorrichtung (20), die ein zweites Positionierungselement (21), das so konfiguriert ist, dass es von dem Ortungsmodul (40) erfasst werden kann, und eine Bohrführung (23) umfasst, die so konfiguriert ist, dass sie eine Bohrachse (32, 32') anzeigt;
- das Ortungsmodul (40), das mindestens einen Sensor umfasst, der eine Technologie des Typs Bildsensor oder Infrarotsensor verwendet, wobei das Ortungsmodul (40) so konfiguriert ist, dass es eine Positionierung des ersten Positionierungselements (13) und des zweiten Positionierungselements (21) in einem dreidimensionalen Raum eines mit dem Ortungsmodul assoziierten Bezugssystems bestimmt (40);
- ein Verarbeitungsmodul (60), das mit dem Ortungsmodul (40) verbunden ist, um die Positionierung des ersten Positionierungselements (13) und des zweiten Positionierungselements (21) aufzunehmen;
- ein Anzeigemodul (30), das mit dem Verarbeitungsmodul (60) verbunden ist und zum Darstellen von Visualisierungselementen (31, 31') geeignet ist, die eine relative Positionierung der Befestigungsöffnung (4) in Bezug auf die durch das Verarbeitungsmodul (60) bestimmte Bohrführung (23) anzeigt;
wobei die Baugruppe **dadurch gekennzeichnet ist, dass** die zweite Vorrichtung (20) ebenfalls mindestens ein Kalibrierungselement (24) umfasst, das so konfiguriert ist, dass es während eines Kalibrierungsschrittes mit der Befestigungsöffnung (4) des Implantats (2) zusammenwirkt, bevor das Implantat (2) in den Knochen (3) eingeführt wird,
**und dadurch, dass** das Verarbeitungmodul (60) für Folgendes ausgelegt ist:
- während der Kalibrierungsphase, Bestimmen einer Positionierung der Befestigungsöffnung (4) in einem Bezugssystem der ersten Vorrichtung (10) anhand der Positionierung des ersten Positionierungselements (13) und des zweiten Positionierungselements (21) sowie anhand mechanischer Maße, die sich auf die Position des mindestens einen Kalibrierungselements (24) der zweiten Vorrichtung (20) in einem Bezugssystem der zweiten Vorrichtung (20) beziehen; und,
- sobald das Implantat (2) in den Knochen (3) eingeführt ist, Bestimmen der relativen Position des Befestigungslochs (4) in Bezug auf die Bohrführung (23) anhand der Position des ersten Positionierungselements (13) und des zweiten Positionierungselements (21), der Positionierung des Befestigungslochs (4) in dem Bezugsssystem der ersten Vorrichtung (10), und mechanischer Maße, die die Position der Bohrführung (23) in dem Bezugssystem der zweiten Vorrichtung (20) angeben.

2. Baugruppe nach Anspruch 1, wobei die Bohrführung (23) mindestens einen Stabilisierungszahn (25) umfasst, der so konfiguriert ist, dass er die Bohrführung (23) auf dem Knochen (3) stabilisiert.

3. Baugruppe nach einem der vorstehenden Ansprüche, wobei das mindestens eine Kalibrierungselement (24) eine Verlängerung ist, die so konfiguriert ist, dass sie in die Befestigungsöffnung (4) eingeführt werden kann.

4. Baugruppe nach Anspruch 3, wobei die Verlängerung aus einem Zapfen und einem Stift ausgewählt ist.

5. Baugruppe nach Anspruch 3 oder 4, wobei sich die Verlängerung der mindestens einen Kalibrierungselements (24) entlang einer im Wesentlichen parallelen Achse zu der Bohrachse (32, 32') der Bohrführung (23) erstreckt.

6. Baugruppe nach einem der vorstehenden Ansprüche, wobei das mindestens eine Kalibrierungselement (24) mindestens zwei parallel verlaufende Kalibrierelemente (24) umfasst.

7. Baugruppe nach einem der vorstehenden Ansprüche, wobei das erste Positionierungselement (13) und/oder das zweite Positionierungselement (21) aus einem Sensor und einer Geometrie ausgewählt sind, wobei die Geometrie ausgewählt ist aus: einem QR-Code, einem Standbild, einem starren Körper, der mindestens eine reflektierende Markierung oder mindestens drei Verlängerungen umfasst, die in drei verschiedenen Richtungen positioniert sind.

8. Baugruppe nach einem der vorstehenden Ansprüche, wobei das Ortungsmodul fest in einem mit dem Anzeigemodul assoziierten Bezugssystem ist.

9. Baugruppe nach einem der vorstehenden Ansprüche, wobei die visuellen Elemente (31, 31') die Bohrachse (32, 32') und eine Bohrrichtlinie (33, 33', 33") zeigen, die so konfiguriert ist, dass sie eine optimale Positionierung eines Bohrwerkzeugs (5) in Bezug auf die Befestigungsöffnung (4) anzeigt.

10. Baugruppe nach einem der vorstehenden Ansprüche, unter Verwendung einer Augmented-Reality-Technologie, bei der das Anzeigemodul dazu geeignet ist, die visuellen Elemente (31') überlagert in dem Sichtfeld eines Benutzers des Hilfssystems darzustellen.

11. Baugruppe nach Anspruch 10, wobei das Anzeigemodul eine Ortungsvorrichtung umfasst, wobei das Hilfssystem ein Abgleichmodul (50) umfasst, das dazu geeignet ist, eine räumliche Beziehung zu bestimmen, die es ermöglicht, geometrische Beschreibungen, die in einem mit dem Ortungsmodul assoziierten Bezugssystem ausgedrückt sind, in geometrische Beschreibungen umzuwandeln, die in einem mit der Ortungsvorrichtung assoziierten Bezugssystem ausgedrückt sind.

## Claims

1. An assembly comprising:
- an implant (2) provided with at least one fastening orifice (4), and
- an assistance system (1) for fastening said surgical implant (2) to a bone (3) by means of a locking part inserted into a bone attachment orifice, which is configured to be positioned facing the fastening orifice (4) of the implant (2),
the assistance system (1) comprising:
- a first device (10) configured to be mechanically coupled to the implant (2) et comprising a first positioning element (13) configured to be detected by a localization module (40);
- a second device (20) comprising a second positioning element (21) configured to be detected by the localization module (40) and a drilling guide (23) configured to indicate a drilling axis (32, 32');
- the localization module (40) that comprises at least one sensor using an image detector or infrared detector technology, said localization module (40) being configured to determine a positioning of the first positioning element (13) and the second positioning element (21) in a three-dimensional space of a reference frame associated with the localization module (40);
- a processing module (60) connected to the localization module (40) for receiving the positioning of the first positioning element (13) and the second positioning element (21);
- a display module (30) connected to the processing module (60) and capable of representing visual elements (31, 31') indicating a relative positioning of the fastening orifice (4) relative to the drilling guide (23) determined by the processing module (60);
the assembly being **characterized in that** the second device (20) also comprises at least one calibration element (24) configured to cooperate with the fastening orifice (4) of the implant (2) during a calibration step, before introducing the implant (2) into the bone (3),
**and in that** the processing module (60) is designed to:
- during the calibration step, determine a positioning of the fastening orifice (4) in a reference frame of the first device (10) from the positioning of the first positioning element (13) and the second positioning element (21) and mechanical dimensions referencing the position of the at least one calibration element (24) of the second device (20) in a reference frame of the second device (20); and to
- after introduction of the implant (2) into the bone (3), determine the relative positioning of the fastening orifice (4) relative to the drilling guide (23) from the positioning of the first positioning element (13) and the second positioning element (21), the positioning of the fastening orifice (4) in the reference frame of the first device (10), and mechanical dimensions specifying the position of the drilling guide (23) in the reference frame of the second device (20).

2. The assembly according to claim 1, wherein the drilling guide (23) comprises at least one stabilization tooth (25) configured to stabilize the drilling guide (23) on the bone (3).

3. The assembly according to any one of the preceding claims, wherein the at least one calibration element (24) is an elongation configured to be introduced into the fastening orifice (4).

4. The assembly according to claim 3, wherein the elongation is selected from a stud and a pin.

5. The assembly according to claim 3 or 4, wherein the elongation of the at least one calibration element (24) extends along an axis substantially parallel to the drilling axis (32, 32') of the drilling guide (23).

6. The assembly according to any one of the preceding claims, wherein the at least one calibration element (24) comprises at least two calibration elements (24) extending in parallel.

7. The assembly according to any one of the preceding claims, wherein the first positioning element (13) and/or the second positioning element (21) are selected from a sensor and a geometry, said geometry being selected from: a QR code, a still image, a rigid body comprising at least one reflective marker or at least three elongations positioned along three different directions.

8. The assembly according to any one of the preceding claims, wherein the localization module is fixed in a reference frame associated with the display module.

9. The assembly according to any one of the preceding claims, wherein the visual elements (31, 31') show the drilling axis (32, 32') and a drilling sight (33, 33', 33") configured to indicate an optimal positioning of a drilling tool (5) relative to the fastening orifice (4).

10. The assembly according to any one of the preceding claims, using an augmented reality technology, wherein the display module is capable of representing the visual elements (31'), in superposition in the visual field of a user of the assistance system.

11. The assembly according to claim 10, wherein the display module comprises a localization device, the assistance system comprising a matching module (50) capable of determining a spatial relationship making it possible to transform geometrical descriptions expressed in a reference frame associated with the localization module, into geometrical descriptions expressed in a reference frame associated with the localization device.
